# EUROPEAN PATENT APPLICATION

(11) **EP 1 431 760 A1**
(43) Date of publication of application: **23.06.2004**
(21) Application number: 02767986.9
(22) Date of filing: 20.09.2002
(51) Int. Cl.: G01N 30/00, G01N 1/10, B01J 39/06, C07C 47/058, C07C 45/79

(54) **PROCESS FOR PRODUCTION OF COLLECTION COLUMNS FOR CARBONYL COMPOUNDS**

(30) Priority: 25.09.2001 JP 2001290843
(71) Applicant: SUMIKA CHEMICAL ANALYSIS SERVICE, LTD., Osaka-shi Osaka 554-8558 (JP)
(72) Inventor: KITASAKA, K. Sumika Chemical Analysis Service Ltd., Osaka-shi, Osaka 554-8558 (JP); SUGIHARA, K. Sumika Chemical Analysis Service Ltd., Osaka-shi, Osaka 554-8558 (JP)
(74) Representative: VOSSIUS & PARTNER
(86) International application number: PCT/JP2002/009664
(87) International publication number: WO 2003/027666

(57) **Abstract**

There is provided a method for producing a sampling tube for carbonyl compound, manifesting low blank value and enabling analysis of carbonyl compounds such as formaldehyde and the like in air or in water with high sensitivity and high accuracy by a simple manner, which comprises the following steps:
contacting a cation exchanger packed in a tube with a hydrophilic solvent or a mixed solvent containing this;
contacting the cation exchanger contacted with a hydrophilic solvent or a mixed solvent containing this with a solution of a compound of the following structural formula (1): 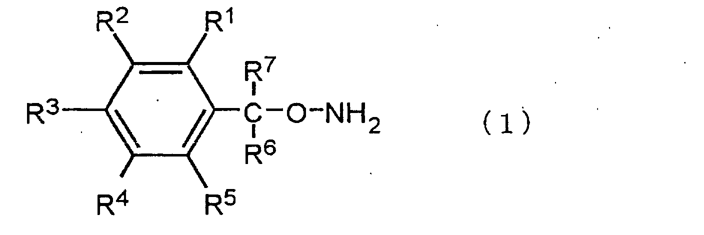 [in the formula, R¹, R², R³, R⁴ and R⁵ each independently represent a hydrogen atom, halogen atom, alkyl group, haloalkyl group, alkoxyl group, haloalkoxyl group, aryl group or haloaryl group, and R⁶ and R⁷ represent a hydrogen atom or alkyl group (wherein, when R⁶ and R⁷ represent a hydrogen atom, at least one of R¹, R², R³, R⁴ and R⁵ is not a fluorine atom)] or a salt thereof, to allow the cation exchanger to support the compound; and
washing the cation exchanger supporting the compound with a hydrophilic solvent or a mixed solvent containing this two or more times.

## Description

### TECHNICAL FIELD

The present invention relates to a method for producing a sampling tube for carbonyl compound used for capturing a carbonyl compound such as formaldehyde.

### BACKGROUND ART

Recently, an influence of volatile organic compounds on environment is regarded as a problem. For example, there is a social problem such as an influence of carbonyl compounds such as formaldehyde and the like diffused from building materials and furniture on housing environment and working environment caused by an airtight structure of houses. For investigating countermeasures against such a problem, it is necessary to first measure accurately the amount of carbonyl compounds in air, and therefore, development of a method which can measure the amount of carbonyl compounds such as formaldehyde and the like in air in room or the like at high sensitivity and at high accuracy, is desired.

Under such situations, the present inventors have developed a sampling tube for carbonyl compound which can measure the amount of carbonyl compounds in air at high sensitivity and at high accuracy, and filed patent applications as "method of production of sampling tube for carbonyl compound"(JP 2001-113180A) and the like. In them, there is disclosed a sampling tube packed with a sampling material for a carbonyl compound characterized in that a reagent capable of converting a carbonyl compound into a derivative thereof is supported on a cation exchanger, and there are exemplified strong acidic cation exchange resins obtained by introducing a sulfonic group or the like into a styrene-based resin such as cross-linked polystyrene, silica gel or the like, as the cation exchanger, and O-(2,3,4,5,6-pentafluorobenzyl)hydroxylamine, as the reagent capable of converting a carbonyl compound to a derivative thereof.

However, an imine derivative of O-2,3,4,5,6-pentafluorobenzyl)hydroxylamine and formaldehyde had a volatility, resultantly sometimes affected correct measurement. For improving this problem, a sampling material for a carbonyl compound using an O-(haloalkoxybenzyl)hydroxylamine compound represented by O-(4-trifluoromethoxybenzyl)hydroxylamine, has been developed and a patent application has been filed as "O-(haloalkoxybenzyl)hydroxylamine and sampling material for carbonyl compound using the same" (Japanese Application No. 2000-392967).

Such a sampling tube for carbonyl compound can be obtained by a method in which a reagent capable of converting a carbonyl compound to a derivative thereof such as O-(2,3,4,5,6-pentafluorobenzyl)hydroxylamine, O-(4-trifluoromethoxybenzyl)hydroxylamine or the like is dissolved in a suitable organic solvent, water or the like, the resulted solution is flown through a tube packed with a cation exchanger, then, an organic solvent, water or the like is removed, or the like. However, in synthesizing of the reagent capable of converting a carbonyl compound to a derivative thereof and flowing of the reagent capable of converting a carbonyl compound to a derivative thereof through the cation exchanger, derivatives with carbonyl compounds are by-produced, and resultantly, if measured using the produced sampling tube for carbonyl compound, the blank value, namely, a measured value, when a solvent hypothesized to contain no carbonyl compound, for example, acetonitrile or the like for liquid chromatography is flown, may increase. Therefore, it was necessary to decrease the blank value in production of such a sampling tube for carbonyl compound.

When O-(2,3,4,5,6-pentafluorobenzyl)hydroxylamine or a salt thereof is used as the reagent capable of converting a carbonyl compound to a derivative thereof, the blank value can be reduced to the range causing no problems by flowing a solution of the reagent capable of converting a carbonyl compound to a derivative thereof in an organic solvent, water or the like through a tube packed with a cation exchanger, then, washing the cation exchanger with a hydrophilic solvent such as acetonitrile, methyl alcohol or the like. However, when a compound of the following structural formula (1): 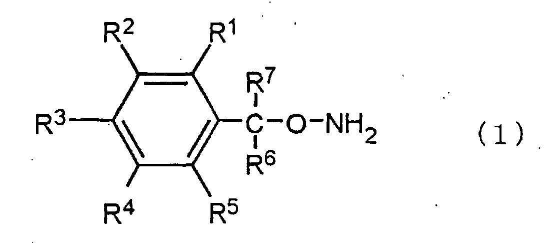 [in the formula, respective R¹, R², R³, R⁴ and R⁵ independently represent a hydrogen atom, a halogen atom, an alkyl group, a haloalkyl group, an alkoxyl group, a halo alkoxyl group, an aryl group or a haloaryl group, and R⁶ and R⁷ represent a hydrogen atom or alkyl group (wherein, when R⁶ and R⁷ represent a hydrogen atom, at least one of R¹, R², R³, R⁴ and R⁵ is not a fluorine atom)], or a salt thereof. For example, when O-(4-trifluoromethoxybenzyl)hydroxylamine is used as the reagent capable of converting a carbonyl compound to a derivative thereof, there are problems that even if a cation exchanger is washed with a hydrophilic solvent such as acetonitrile, methyl alcohol or the like, the blank value sometimes increases, therefore, the accurate concentration of a carbonyl compound can not be evaluated with the produced sampling tube.

The present inventors have studied to develop a method capable of producing a sampling tube for carbonyl compound having a blank value reduced to the range causing no problems also when a compound of the structural formula (1) or a salt thereof is used as the reagent capable of converting a carbonyl compound to a derivative thereof, and resultantly found that the blank value can be decreases to the range causing no problems for analysis by washing a tube packed with a cation exchanger after contact with a solution of a reagent capable of converting a carbonyl compound to a derivative thereof, with a hydrophilic solvent such as acetonitrile, methyl alcohol or the like two or more times, leading to completion of the present invention.

### DISCLOSURE OF THE INVENTION

Namely, the present invention provides a method for producing a sampling tube for carbonyl compound, which comprises the following steps of:
contacting a cation exchanger packed in a tube with a hydrophilic solvent or a mixed solvent containing this;
contacting the cation exchanger contacted with the hydrophilic solvent or the mixed solvent containing this with a solution of a compound represented by the structural formula (1) or a salt thereof, to allow the cation exchanger to support the compound thereon; and
washing the cation exchanger supporting the compound with a hydrophilic solvent or a mixed solvent containing this two or more times.

### BEST MODE FOR CARRYING OUT THE INVENTION

The cation exchanger used in the present invention means a material obtained by introducing a cation exchange group, namely, a group of which a counter ion is a cation, into a polymer substrate such as a resin, cellulose, silica gel or the like. As the cation exchange group, when the polymer substrate is a resin, namely, when the cation exchanger is a cation exchange resin, there are exemplified a sulfonic group and carboxyl group, however, in the present invention, a strong acidic cation exchange resin of sulfonic acid type is preferable. When a polymer substrate is made of a resin, the resin is preferably a styrene-based rein such as cross-linked polystyrene, and preferable is for example a copolymer of styrene and divinylbenzene, and other examples may include, but not particularly limited to, acrylic resins, methacrylic resins and the like. When a polymer substrate is made of cellulose, namely, in the case of a cation exchanger obtained by introducing a cation exchange group into cellulose, examples of the cation exchange group include a sulfoethyl group, phosphomethyl group, phosphoric acid group, carboxymethyl group and the like, and in the present invention, preferable is a strong acidic exchanger into which a sulfoethyl group or the like is introduced. Further, when a polymer substrate is silica gel, examples of the cation exchange group include groups of benzenesulfonic acid, propylsulfonic acid, carboxylic acid and the like, and in the present invention, a strong acidic exchanger into which a benzenesulfonic group or the like is introduced is preferable. As the polymer substrate, styrene-based resins and silica gel are preferable.

The method for producing a sampling tube for carbonyl compound has the above-described three steps, and the first step is a step in which a cation exchanger packed in a tube is contacted with a hydrophilic solvent or a mixed solvent containing this. The cation exchanger is activated by contact with the hydrophilic solvent or the mixed solvent containing this, and resultantly capable of supporting a reagent capable of converting a carbonyl compound to a derivative thereof successfully by contact with the reagent capable of converting a carbonyl compound to a derivative thereof.

The tube to be packed with a cation exchanger is not particularly restricted in its material, size, form and the like, and usually, is a glass tube, plastic tube or the like, and those having an internal diameter of about 3 to about 15 mm and a length of about 1 to about 10 cm are often used. It may have a form of porous tube.

As the hydrophilic solvent used for activation of a cation exchanger, there are exemplified lower aliphatic nitriles, lower alcohols, lower aliphatic ethers or lower cyclic ethers. In the present specification, the lower aliphatic nitrile means an aliphatic nitrile having about 6 or less carbon atoms, for example, acetonitrile and the like, the lower alcohol means an alcohol having about 5 or less carbon atoms, for example, methyl alcohol and ethyl alcohol, the lower aliphatic ether means an aliphatic ether having about 10 or less carbon atoms, and the lower cyclic ether means a cyclic ether having about 6 or less carbon atoms, for example, tetrahydrofuran. Of these hydrophilic solvents, acetonitrile and methyl alcohol are preferable, and acetonitrile is particularly preferable. The above-mentioned solvents may be used singly or in admixture of two or more. In the range in which an effect of activating a cation exchanger is obtained, it may be mixed with other liquid. As the other liquid, water and the like are listed. For example, a mixed liquid of acetonitrile/water is used, and the mixing ratio in this case is preferably in the range of 30/70 to 100/0 in volume ratio.

The method for contacting a cation exchanger packed in a tube with a hydrophilic solvent or a mixed solvent containing this may be a method in which the hydrophilic solvent or the mixed solvent containing this is flown through the tube packed with the cation exchanger. However, from the standpoint of productivity, a method in which the tube packed with the cation exchanger is immersed into the hydrophilic solvent or the mixture containing this, is preferable.

Immersion of the tube packed with the cation exchanger into the hydrophilic solvent or the mixture containing this (hereinafter, referred to as "immersion liquid") is usually conducted by placing the immersion liquid into a vessel of size capable of immersing the whole body of the tube packed with the cation exchanger, and immersing therein sufficiently the whole body of the tube packed with the cation exchanger. The immersion temperature and time vary depending on the kinds of the cation exchanger and immersion liquid, and usually, immersion is conducted at a temperature in a tube of 0 to 50°C for 10 to 100 minutes, preferably at 10 to 30°C for 30 to 100 minutes. The amount of immersion liquid may be that capable of sufficiently immersing the whole body of the tube packed with the cation exchanger. Accordingly, when the amount of immersion liquid is increased, the number of tubes which can be immersed in one time can be increased and the productivity is improved.

Such immersion may be conducted two or more times. Namely, it may also be possible that a tube packed with a cation exchanger is immersed into an immersion liquid, then, the tube is removed out of the immersion liquid, and the tube is further immersed into the same or different kind of the immersion liquid, and by this method, the cation exchanger can be further sufficiently activated.

In immersing the tube packed with the cation exchanger into the immersion liquid, it is preferable to deaerate the cation exchanger.

Though the deaeration method is not particularly restricted, deaeration is usually conducted by applying reduced pressure on an immersion liquid immersing a tube packed with a cation exchanger. Preferable conditions for pressure reduction vary depending on the kind of the immersion liquid, and the like, and when acetonitrile or a mixed liquid containing it is used, the pressure is usually 870 hPa or lower. The pressure reducing time varies depending on the number of tubes, the amount of the hydrophilic solvent or mixed liquid thereof, and the like, and usually, pressure reduction is conducted for further about 30 minutes after disappearance of generation of bubbles from the tube. In this deaeration, irradiation of ultrasonic wave may be effected.

The tube packed with thus activated cation exchanger is contacted with a solution of a reagent capable of converting a carbonyl compound to a derivative thereof, the reagent being represented by the structural formula (1) or a salt thereof, to allow the reagent capable of converting a carbonyl compound to a derivative thereof to be supported on the cation exchanger.

As the compound represented by the structural formula (1), O-(haloalkoxybenzyl)hydroxylamine compounds are exemplified. Examples of the O-(haloalkoxybenzyl)hydroxylamine compound include O-(4-trifluoromethoxybenzyl)hydroxylamine, O-[3,5-bis(trifluoromethyl)benzyl]hydroxylamine and O-(4-trifluoromethylbenzyl)hydroxylamine, and among them, O-(4-trifluoromethoxybenzyl)hydroxylamine or its hydrochloride is preferably mentioned.

The amount of the reagent capable of converting a carbonyl compound to a derivative thereof supported on the cation exchanger is usually from about 0.05 to about 2 mg per 100 mg of the cation exchanger.

The method of contacting the cation exchanger packed in a tube with a solution of the reagent capable of converting a carbonyl compound to a derivative thereof is not particularly restricted.

For example, the contact can be conducted by flowing the solution of the reagent capable of converting a carbonyl compound to a derivative thereof in an organic solvent through the tube packed with the cation exchanger activated as described above, or by immersing the tube packed with the cation exchanger into the solution of the reagent capable of converting a carbonyl compound to a derivative thereof in the organic solvent.

After the contact with the solution of the reagent capable of converting a carbonyl compound to a derivative thereof in the organic solvent, the cation exchanger is further washed with an organic solvent, then, the organic solvent is removed.

Here, examples of the organic solvent include aliphatic hydrocarbons such as hexane, alicyclic hydrocarbons such as cyclohexane, aromatic hydrocarbons such as benzene and toluene, alcohols such as methyl alcohol and ethyl alcohol, and acetonitrile. Removal of the organic solvent is usually conducted under a condition of reduced pressure.

The reagent capable of converting a carbonyl compound to a derivative thereof represented by the structural formula (1) is often synthesized in the form of hydrochloride. In the case of supporting on a weak acidic cation exchanger, a reagent capable of converting a carbonyl compound to a derivative thereof previously neutralized with an alkali is dissolved in an organic solvent before use, while in the case of supporting on a strong acidic cation exchanger, a hydrochloride of a reagent capable of converting a carbonyl compound to a derivative thereof can be used as it is.

Instead of the solution of the reagent capable of converting a carbonyl compound to a derivative thereof in the organic solvent, an aqueous solution of the reagent capable of converting a carbonyl compound to a derivative thereof can also be used. For example, after an aqueous solution of the reagent capable of converting a carbonyl compound to a derivative thereof is flown through a tube packed with the cation exchanger or after immersing a tube packed with the cation exchanger into the aqueous solution of the reagent capable of converting a carbonyl compound to a derivative thereof, it can be attained by removing water in the tube.

So far as dissolution of the reagent capable of converting a carbonyl compound to a derivative thereof, instead of the solution of the reagent capable of converting a carbonyl compound to a derivative thereof in an organic solvent and an aqueous solution of the reagent, a mixed liquid of an organic solvent and water can also be used. For example, it is also possible to use a mixed liquid of acetonitrile and water.

The cation exchanger thus contacted with the reagent capable of converting a carbonyl compound to a derivative thereof to support this compound is washed two or more times with a hydrophilic solvent or a mixed solvent containing this.

As the hydrophilic solvent used for this washing, a lower aliphatic nitrile, a lower alcohol, a lower aliphatic ether or a lower cyclic ether is exemplified. Among these hydrophilic solvents, acetonitrile and methyl alcohol are preferable, and particularly, acetonitrile containing no carbonyl compound such as formaldehyde is preferable. The above-mentioned washing solvents may be used each singly or in admixture of two or more. Further, mixing with another liquid is also permissible so far as an effect of washing after contact with the reagent capable of converting a carbonyl compound to a derivative thereof is obtained. As the other liquid, water or the like is mentioned. Particularly, a mixed solvent of a hydrophilic solvent and water, for example, a mixed liquid of acetonitrile/water is preferably used. The mixing ratio of acetonitrile/water in this case is preferably in the range from 30/70 to 100/0 in volume ratio. The hydrophilic solvent used in this washing may be the same as or different from the solvent used for activation of the cation exchanger.

Washing with the hydrophilic solvent or mixed solvent containing this after contact of the tube packed with the cation exchanger with a solution of the reagent capable of converting a carbonyl compound to a derivative thereof is required to be conducted twice or more. When less than twice, the blank value cannot be sufficiently decreased. In usual case, a washing frequency of two is sufficient, and the blank value does not significantly decrease even if washing is conducted three times or more.

Washing of the cation exchanger supporting the reagent capable of converting a carbonyl compound to a derivative thereof with a hydrophilic solvent or a mixed solvent containing this, is conducted according to a method of immersing the tube packed with the cation exchanger in the hydrophilic solvent or the mixed solvent containing this, a method of flowing the hydrophilic solvent or the mixed solvent containing this through the tube packed with the cation exchanger, or the like. For sufficiently decreasing the blank value, the method of flowing the hydrophilic solvent or the mixed solvent containing this, is a preferable. In the method of immersing in the hydrophilic solvent or mixed solvent containing this, it is necessary to use a large amount of the hydrophilic solvent or mixed solvent containing this for obtaining sufficient washing efficiency. The amount of the hydrophilic solvent or mixed solvent containing this necessary in this case is usually from 5 to 100-fold volume of the cation exchanger.

When the hydrophilic solvent or mixed solvent containing this is flown through the tube packed with the cation exchanger, the temperature and amount of washing liquid in washing vary depending on the kinds of the cation exchanger and washing liquid, and usually, the temperature is from 0 to 50°C and the amount is 1 to 5 ml per one time of flowing per 500 ml of the cation exchanger.

When the hydrophilic solvent or mixed solvent containing this is flown through the tube packed with the cation exchanger, it is preferably conducted under an atmosphere containing no carbonyl compound, for example, under a nitrogen atmosphere. The solvent used for washing is removed after washing, thereafter, the tube is preferably dried.

The sampling tube for carbonyl compound obtained as described above can be used for determination of the amount of a carbonyl compound in air or in water.

When this sampling tube for carbonyl compound is used for the determination of a carbonyl compound in air, usually, a pump is connected to the sampling tube for carbonyl compound and samples in air are collected. For example, when the sampling tube for carbonyl compound has an internal diameter of about 3 to about 15 mm and a length of about 1 to about 10 cm, the aspiration speed of the pump is preferably about 0.01 to about 1.5 l/min. When an amine compound such as O-(4-trifluoromethoxybenzyl)hydroxylamine or the like is used as the reagent capable of converting a carbonyl compound to a derivative thereof, a carbonyl compound in thus aspired sample is converted into an imine compound in the sampling tube, therefore, the produced imine compound is eluted with an organic solvent, for example, an aliphatic hydrocarbon such as hexane, an alicyclic hydrocarbon such as cyclohexane, an aromatic hydrocarbon such as benzene or toluene, an alcohol such as methyl alcohol or ethyl alcohol, or a nitrile such as acetonitrile. By analyzing this elution liquid by liquid chromatography, gas chromatography or the like, quantitative analysis of a carbonyl compound in air can be conducted. Particularly, when analysis is conducted by a capillary GC/MS method or the like, a more accurate analysis becomes possible, desirably.

The present invention will be illustrated more specifically by examples below, but the scope of the invention is not limited to these examples.

### Example 1

### (Activation of cation exchanger)

In a 1000 ml beaker is placed 800 ml of acetonitrile (guaranteed grade reagent) and 120 tubes of BOND ELUTE JR [solid phase extraction column manufactured by Varian Ltd., packed with 500 mg of benzene sulfonic acid ion exchanger (SCX), hereinafter referred to as SCX], and allowed to stand still for 20 minutes. After allowing to stand still, SCX is taken out, and transferred to a beaker containing 800 ml of mixed liquid (50/50 volume ratio) of acetonitrile(guaranteed grade reagent) and purified water previously deaerated by ultrasonic wave. This beaker was placed under a reduced pressure of 130 to 400 hPa for 5 minutes to deaeration. Because of floating of SCX, the pressure was once returned to normal pressure, and after precipitation of SCX, the pressure was again reduced to 130 to 400 hPa.

The beaker was irradiated with an ultrasonic wave of 40 kHz by an ultrasonic wave washing machine UT-105 (manufactured by Sharp Corporation) for 5 minutes, and after the irradiation, the beaker was placed under a reduced pressure of 130 to 400 hPa for further 90 minutes for deaeration.

### (Supporting of reagent capable of converting a carbonyl compound to derivative thereof)

Thereafter, the pressure was returned to normal pressure and the beaker was allowed to stand still for additional 30 minutes, then, SCX was taken out, and this SCX was fixed to an aspiration manifold. Through SCX fixed to the aspiration manifold, 4 ml of a solution obtained by dissolving a predetermined amount (2 to 10 mg, shown as contact amount in Table 1) of O-(4-trifluoromethoxybenzyl)hydroxylamine hydrochloride in mixed liquid (50/50 volume ratio) of acetonitrile (guaranteed grade reagent) and distilled water was flown, then, SCX was placed under a nitrogen atmosphere for 30 minutes to substitute air in SCX with nitrogen.

### (Washing step)

Thereafter; SCX fixed to the aspiration manifold was washed once to three times (shown in Table 1) under a nitrogen atmosphere with acetonitrile(guaranteed grade reagent) in an amount of 5 ml per one washing, then, pressure-reduced (60 to 140 hPa) for 5 minutes and further, dried under reduced pressure (2 to 14 hPa) for 4.0 hours by a vacuum pump. Thus obtained SCX was closely capped on its both ends with lure plugs to obtain a sampling tube for carbonyl compound.

### (Measurement of blank value)

The blank value (amount of an imine derivative of O-(4-trifluoromethoxybenzyl)hydroxylamine with formaldehyde)of the sampling tube for carbonyl compound produced by the above-mentioned method was measured by flowing acetonitrile through the cartridge, keeping the amount of the elution liquid constant at 5 ml, obtaining 1 µl of the sample therefrom and subjecting it to analysis using GC/MS. The results are shown in Table 1.

**Table 1**

| Contact amount (mg) | Blank value (µg)* | | |
|---|---|---|---|
| | Once | Twice | Three times |
| 2 | 0.394 | 0.075 | 0.052 |
| 3 | 0.376 | 0.083 | 0.061 |
| 4 | 0.363 | 0.085 | 0.080 |
| 8 | 0.361 | 0.082 | 0.082 |
| 10 | 0.341 | 0.096 | 0.071 |

| | | | |
|---|---|---|---|
| * Formaldehyde-reduced amount | | | |

As apparent from the results in Table 1, the blank value is still high by washing once, however, the blank value significantly decreases to level causing scarce problems by washing twice. By washing three times, the blank value decreases while there is no remarkable difference from the case of washing twice.

According to the method for producing a sampling tube for carbonyl compound, a sampling tube for carbonyl compound of low blank value can be produced. A sampling tube for carbonyl compound obtained by the method of the present invention enables analysis of carbonyl compounds such as formaldehyde and the like in air or in water with high sensitivity and high accuracy by a simple manner. For example, it enables microanalysis of carbonyl compounds such as formaldehyde and the like in air indoor and outdoor, and can be used also for measurement of aldehydes and the like not only in working environments but also in residences.

## Claims

1. A method for producing a sampling tube for carbonyl compound, which comprises the following steps of:
contacting a cation exchanger packed in a tube with a hydrophilic solvent or a mixed solvent containing this;
contacting the cation exchanger contacted with the hydrophilic solvent or the mixed solvent containing this with a solution of a compound represented by the structural formula (1): 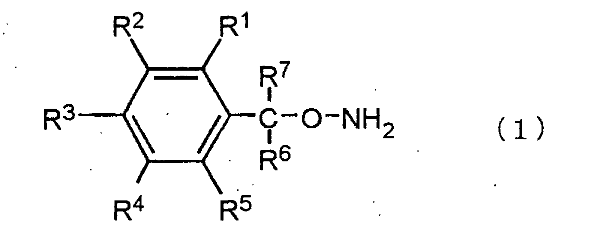 wherein, respective R¹, R², R³, R⁴ and R⁵ independently represent a hydrogen atom, a halogen atom, an alkyl group, a haloalkyl group , an alkoxyl group, a haloalkoxyl group, an aryl group or a haloaryl group, and R⁶ and R⁷ represent a hydrogen atom or alkyl group (wherein, when R⁶ and R⁷ represent a hydrogen atom, at least one of R¹, R², R³, R⁴ and R⁵ is not a fluorine atom)], or a salt thereof, to allow the cation exchanger to support the compound; and
washing the cation exchanger holding the compound with a hydrophilic solvent or a mixed solvent containing this two or more times.

2. The method according to Claim 1, wherein washing with a hydrophilic solvent or a mixed solvent containing this is conducted twice.

3. The method according to Claim 1 or 2, wherein the hydrophilic solvent used for washing the cation exchanger is a lower aliphatic nitrile, a lower alcohol, a lower aliphatic ether or a lower cyclic ether.

4. The method according to Claim 3, wherein the hydrophilic solvent is acetonitrile or methyl alcohol.

5. The method according to Claim 4, wherein the hydrophilic solvent is acetonitrile.

6. The method according to any one of Claims 1 to 5, wherein the compound represented by the structural formula (1) is an O-(haloalkoxybenzyl)hydroxylamine compound.

7. The method according to Claims 6, wherein the O-(haloalkoxybenzyl)hydroxylamine compound is O-(4-trifluoromethoxybenzyl)hydroxylamine.

8. The method according to any one of Claims 1 to 7, wherein the cation exchanger is a strong acidic cation exchanger.

9. The method according to any of Claims 1 to 8, wherein the contact of the cation exchanger packed in the tube with the hydrophilic solvent or the mixed solvent containing this is conducted by immersing the tube packed with the cation exchanger into a hydrophilic solvent or a mixed solvent containing this.

10. The method according to any one of Claims 1 to 9, wherein the contact of a cation exchanger with the solution of the compound represented by the structural formula (1) or the salt thereof is conducted by flowing a solution of the compound represented by the structural formula (1) or salt thereof in an organic solvent, water or a mixed solvent thereof through the tube packed with the cation exchanger, or by immersing the tube packed with the cation exchanger into a solution represented by the compound of the structural formula (1) or salt thereof in an organic solvent, water or a mixed solvent.

11. The method according to any one of Claims 1 to 10, wherein the process of washing with a hydrophilic solvent or a mixed solvent containing this is conducted by flowing a hydrophilic solvent or a mixed solvent containing this through a tube packed with a cation exchanger.

12. The method according to Claim 11, wherein the hydrophilic solvent or mixed solvent containing this is a mixed solvent containing a hydrophilic solvent and water.

13. The method according to Claim 11 or 12, wherein the temperature at which the hydrophilic solvent or the mixed solvent containing this is flown through the tube packed with the cation exchanger is from 0 to 50°C, and the amount of the hydrophilic solvent or the mixed solvent containing this is from 1 to 5 ml per one time of flowing per 500 mg of the cation exchanger.
